(19) Europäisches Patentamt / European Patent Office / Office européen des brevets

(11) EP 1 472 240 B1

(12) # EUROPÄISCHE PATENTSCHRIFT

(45) Veröffentlichungstag und Bekanntmachung des Hinweises auf die Patenterteilung:
**05.05.2010 Patentblatt 2010/18**

(21) Anmeldenummer: **03700811.7**

(22) Anmeldetag: **20.01.2003**

(51) Int Cl.:
***C07D 307/77*** *(2006.01)* ***C12P 17/04*** *(2006.01)*
***C12N 1/14*** *(2006.01)* ***A61K 31/34*** *(2006.01)*
***A61P 25/00*** *(2006.01)*

(86) Internationale Anmeldenummer:
**PCT/EP2003/000487**

(87) Internationale Veröffentlichungsnummer:
**WO 2003/064405 (07.08.2003 Gazette 2003/32)**

(54) **DRECHSLERANOL-DERIVATE, VERFAHREN ZU DEREN HERSTELLUNG UND DEREN VERWENDUNG**

DRECHSLERA ANOLE DERIVATIVES, METHOD FOR THE PRODUCTION AND USE THEREOF

DERIVES D'ANOLES DE DRECHSLERA, PROCEDE DE PRODUCTION ET UTILISATION DESDITS DERIVES

(84) Benannte Vertragsstaaten:
**AT BE BG CH CY CZ DE DK EE ES FI FR GB GR HU IE IT LI LU MC NL PT SE SI SK TR**

(30) Priorität: **29.01.2002 DE 10203557**

(43) Veröffentlichungstag der Anmeldung:
**03.11.2004 Patentblatt 2004/45**

(73) Patentinhaber: **Sanofi-Aventis Deutschland GmbH**
**65929 Frankfurt am Main (DE)**

(72) Erfinder:
- **EDER, Claudia**
**65719 Hofheim (DE)**
- **KURZ, Michael**
**65719 Hofheim (DE)**
- **TOTI, Luigi**
**65239 Hochheim (DE)**

(56) Entgegenhaltungen:
**EP-A- 0 557 939**

- **CHEMICAL ABSTRACTS, vol. 97, no. 13, 27. September 1982 (1982-09-27) Columbus, Ohio, US; abstract no. 107285, SHATLA M. N. ET AL: "Studies on Drechslera australiensis and Alternaria alternata leaf pot of soybean in Egypt" XP002238333 & MONOUFEIA J. AGRIC.RES., Nr. 3, 1980, Seiten 35-51,**
- **W. COCKER ET AL: "The elimination of non-angular alkyl groups in aromatisation reactions" JOURNAL OF THE CHEMICAL SOCIETY., 1953, Seiten 2355-2362, XP002238332 LETCHWORTH GB**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue, Drechsleranole genannte Verbindungen, die von dem Mikroorganismus *Drechslera australiensis.* ST 003360, DSM 14093, oder einem taxonomisch nicht näher bestimmten Pilz ST 004112, DSM 14524, während der Fermentation gebildet werden, ein Verfahren zur Herstellung dieser Verbindungen und ihre Verwendung zur Hestellung von Arzneimitteln, insbesondere zur Behandlung und/oder Prophylaxe von degenerativen Neuropathien, beispielsweise der Alzheimer'schen Krankheit, oder psychischer Erkrankungen, wie Depression, Schlafstörungen oder saisonal bedingter affektiver Störungen.

Bei der Alzheimer-Krankheit handelt es sich um eine neuropsychiatrische Erkrankung, die vorwiegend bei älteren Menschen auftritt. Die Erkrankung manifestiert sich in einem Symptomenkomplex, der Gedächtnisstörungen, herabgesetzte Merkfähigkeit, Orientierungsstörungen, Sprachstörungen, Störungen des gezielten Denken usw. einschließt. Bei Alzheimer-Patienten findet man charakteristische neurohistologische Veränderungen im Gehirn, wie beispielsweise die Ablagerungen sogenannter amyloider Plaques sowie auch eine Degeneration der Neurofibrillen in den Nervenzellen (sog. "fibrilläre Bündel"). Diese Veränderungen sind zwar charakteristisch, jedoch unspezifisch, da sie in geringerem Ausmaß auch beim normalen Alterungsprozess auftreten.

Derzeit ist keine kausale, sondern nur eine symptomatische Behandlung möglich. Bisher gibt es Medikamente, die den Verlauf der Krankheit lediglich verzögern, sie aber nicht heilen können. Den wichtigsten therapeutischen Ansatz bietet derzeit die Gruppe der cerebralen Acetylcholinesterase-Hemmer (Tacrin®, Donepezil®, Rivastigmin®, Galantamin®), da für die gedächtnisrelevanten Strukturen, die bei Alzheimer in besonderem Maße beeinträchtigt sind, die cholinerge Signalübertragung eine große Rolle spielt. Die Medikamente können allerdings nur bei leichtem und mittlerem Krankheitsstadium eingesetzt werden. Sie erhöhen die Konzentration von Acetylcholin in den informationsübertragenden Synapsen des Gehirns. Bei zu starker Schädigung der Neuronen, also im Spätstadium der Krankheit, sind sie nicht mehr wirksam. Weitere Substanzen, deren Einsatz überprüft wird sind Östrogene, nichtsteroidale Analgetika, Antioxidantien und Nerven-Wachstumsfaktoren (NGF).

Man schätzt, daß es gegenwärtig etwa eine Million Menschen in der Bundesrepublik Deutschland gibt, die an der Alzheimer-Krankheit leiden. Diese Zahl wird vermutlich aufgrund der steigenden Lebenserwartung der Bevölkerung in den nächsten Jahren noch weiter ansteigen. Daher sind dringend neue Substanzen zur Behandlung dieser Erkrankung notwendig.

Bei der Gruppe der c-Jun N-terminalen Kinasen (JNKs) handelt es sich um Proteinkinasen, die durch oxidativen Stress aktiviert werden. Bisher ist bekannt, dass nur JNK-3 (im Gegensatz zu JNK-1 und JNK-2) in den Neuronen des menschlichen Gehirns exprimiert wird. Es gibt Hinweise darauf, dass die JNKs einen Einfluss auf den Zelltod haben. Dieser Zelltod (oder Apoptose) ist wahrscheinlich der ursächliche Mechanismus des Absterbens der Neuronen im Gehirn von Alzheimer-Patienten (Kumagae et al., Mol. Brain Res. (1999), 67(1), 10-7). Die Aktivierung von c-Jun N-terminalen Kinasen ist ein Schritt innerhalb dieses Mechanismus. Eine Inhibierung an dieser Stelle sollte also die Apoptose verhindern, und damit dem Entstehen der Alzheimer-Erkrankung entgegenwirken und ihr Fortschreiten aufhalten.

Ein weiterer Aspekt der vorliegenden Erfindung ist die Behandlung und/oder Prophylaxe von psychischen Erkrankungen. Zirkadiane Rhythmen werden durch endogene Zeitgeber erzeugt, die in den unterschiedlichsten Organismen vorhanden sind. Die zirkadiane Uhr ist wichtig für die Aufrechterhaltung des biologischen Rhythmus. Sie ist selbsterhaltend und konstant, auch bei totaler Dunkelheit, aber sie kann durch äußere Signale, wie z.B. Änderungen der Helligkeit oder der Temperatur synchronisiert werden. Die innere Uhr kontrolliert die täglichen Fluktuationen des Verhaltens, der Aktivität, der Nahrungsaufnahme, des Schlaf/Wachzykluses genauso wie physiologische Änderungen wie beispielsweise Hormonsekretion und Änderungen der Körpertemperatur (Keesler et al., Neuroreport (2000), 11(5), 951-955).

Period (PER) ist ein zentrales Protein dieser zirkadianen Uhr, das täglichen Schwankungen bezüglich seiner Konzentration bzw. seines Phosphorylierungszustandes unterliegt. Die Phosphorylierung des humanen PER1 (hPER1) durch das Enzym human casein kinase 1 epsilon (hCK1ε) ruft eine Abnahme der Proteinstabilität bei hPER1 hervor. Phosphoryliertes hPER1 hat eine Halbwertszeit von ungefähr 12 Stunden, wohingegen nicht phosphoryliertes hPER1 in Zellen länger als 24 Stunden stabil bleibt. Eine Beeinflussung dieses zentralen Proteins hPER1 ist klinisch von Bedeutung vor allem bei Krankheiten, die mit einer Störung der inneren Uhr in Zusammenhang gebracht werden, wie beispielsweise Depression (Souetre E. et al., Annales medico-physiologiques, 1985, 143(9), 845-870), Schlafstörungen oder saisonal bedingter affektiver Störungen. Für die Behandlung der Depression stehen bisher Monoaminoxidase-Hemmer und Hemmstoffe der Wiederaufnahme von Noradrenalin und/oder Serotonin ins Axoplasma (z.B. trizyklische Antidepressiva) zur Verfügung, wobei der exakte Wirkmechanismus bisher nicht geklärt ist. Mit einem Hemmstoff der hCK1ε hätte man ein neues Wirkprinzip für die Behandlung von psychischen Erkrankungen zur Verfügung, wie beispielsweise von Schlafstörungen, saisonal bedingten affektiven Störungen und inbesondere von Depression.

W. Cocker et al (Journal of the Chemical Society, 1953, 2355-2362) beschreiben Dinaphthofuran das optional di-, tetra- und hexa-Methyl-substituiert sein kann.

Die Erfindung betrifft eine Verbindung der Formel (I)

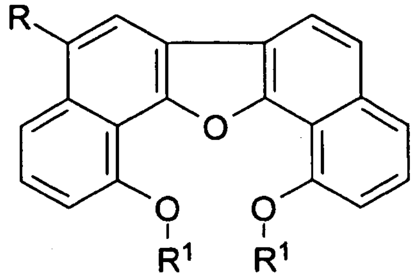

(I)

wobei

R H, oder eine Gruppe der Formel $-(CH(OR^2))_5-CH_2-OR^2$, und

$R^1$ und $R^2$ unabhängig voneinander

1.0 H oder

2.0 eine $C_1$-$C_6$-Alkyl-, $C_2$-$C_6$-Alkenyl-, $C_2$-$C_6$-Alkinyl- oder $C_5$-$C_{10}$-Aryl-Gruppe bedeuten, worin Alkyl, Alkenyl und Alkinyl geradkettig oder verzweigt sind, und worin die Gruppen gegebenenfalls ein- oder zweifach substituiert sind durch:

2.1-OH,
2.2=O,
2.3-O-$C_1$-$C_6$-Alkyl, worin Alkyl geradkettig oder verzweigt ist,
2.4-O-$C_2$-$C_6$-Alkenyl, worin Alkenyl geradkettig oder verzweigt ist,
2.5-Aryl,
2.6-NH-$C_1$-$C_6$-Alkyl, worin Alkyl geradkettig oder verzweigt ist,
2.7-NH-$C_2$-$C_6$-Alkenyl, worin Alkenyl geradkettig oder verzweigt ist,
2.8-$NH_2$ oder
2.9 Halogen,
worin die Substituenten 2.3 bis 2.7 weiter substituiert sein können durch -CN, - Amid oder -Oxim-Funktionen,

oder eine stereoisomere Form der Verbindung der Formel (I) oder ein physiologisch verträgliches Salz der Verbindung der Formel (I) oder ein physiologisch verträgliches Salz einer stereoisomeren Form einer Verbindung der Formel (I).

$C_1$-$C_6$-Alkyl bedeutet ein geradkettiges oder verzweigtes Alkyl mit 1 bis 6 C-Atomen, bevorzugt mit 1 bis 4 C-Atomen, z.B. Methyl, Ethyl, i-Propyl, tert.Butyl und Hexyl.

$C_2$-$C_6$-Alkenyl bedeutet ein geradkettiges oder verzweigtes Alkenyl mit 2 bis 6 C-Atomen, das einfach, zweifach oder dreifach ungesättigt ist, z.B. Allyl, Crotyl, 1-Propenyl, Penta-1,3-dienyl und Pentenyl.

$C_2$-$C_6$-Alkinyl bedeutet ein geradkettiges oder verzweigtes Alkinyl mit 2 bis 6 C-Atomen, das einfach oder zweifach ungesättigt ist, z.B. Propinyl, Butinyl und Pentinyl.

$C_5$-$C_{10}$-Aryl bedeutet eine Arylgruppe mit 5 bis 10 C-Atomen, z.B. Phenyl, Benzyl oder 1- oder 2- Naphthyl, die auch noch substituiert sein kann, beispielsweise durch Halogen, wie Chlor, Brom, Fluor, durch Alkyl mit 1-4 C-Atomen, vorzugsweise Methyl-, durch Hydroxy, durch Alkoxy mit 1-4 C Atomen, insbesondere Methoxy oder durch Trifluormethyl stehen.

Vorzugsweise betrifft die Erfindung eine Verbindung der Formel (I), in der R H oder eine Gruppe der Formel $-(CH(OR^2))_5-CH_2-OR^2$ ist, $R^1$ und $R^2$ unabhängig voneinander H oder $C_1$-$C_6$-Alkyl sind, oder eine stereoisomere Form und/oder ein physiologisch verträgliches Salz dieser bevorzugten Verbindung.

Besonders bevorzugt betrifft die Erfindung eine Verbindung der Formel (I), in der R eine Gruppe der Formel $-(CH(OR^2))_5-CH_2-OR^2$, und $R^1$ und $R^2$ H bedeuten, oder eine stereoisomere Form und/oder ein physiologisch verträgliches Salz dieser besonders bevorzugten Verbindung. Eine solche Verbindung wird durch Formel (II) beschrieben:

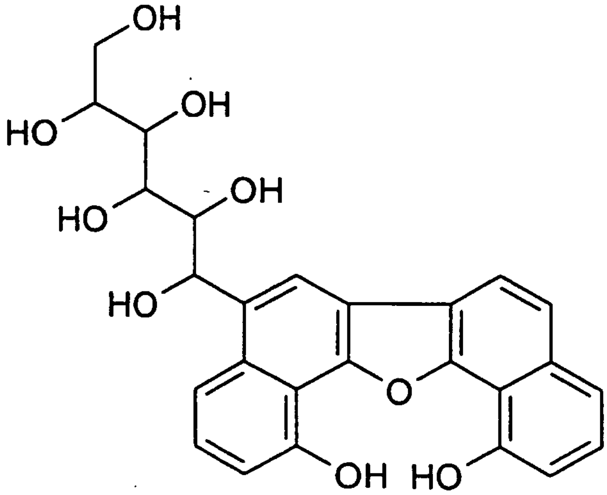

(II)

Ein weiterer besonders bevorzugter Gegenstand der Erfindung ist eine Verbindung der Formel (I), in der R und $R^1$ H bedeuten, oder eine stereoisomere Form und/oder ein physiologisch verträgliches Salz dieser Verbindung. Eine solche Verbindung wird durch Formel (III) beschrieben:

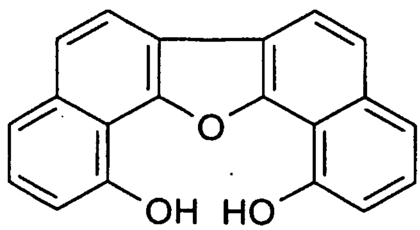

(III)

Chiralitätszentren in der Verbindungen der Formeln (I) und (II) können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft weiterhin offensichtliche chemische Äquivalente der-Verbindungen der Formeln (I), (II) oder (III).

Offensichtliche chemische Äquivalente der erfindungsgemäßen Verbindungen sind Verbindungen, die die gleiche Wirksamkeit wie die erfindungsgemäßen Verbindungen haben und einen geringfügigen chemischen Unterschied aufweisen oder sich unter milden Bedingungen in die erfindungsgemäßen Verbindungen umwandeln. Zu den genannten Äquivalenten gehören z.B. Ether, Ester, Reduktionsprodukte und Komplexe der erfindungsgemäßen Verbindungen.

Beispielsweise kann eine oder mehrere Hydroxygruppen der Verbindungen der Formel (I), (II) oder (III), beispielsweise mit einem $C_1$-$C_6$-Alkohol unter Säurezusatz verethert werden, oder mit beispielsweise einer aktivierten Säure, zum Beispiel Säurechloride oder andere Säure-Derivate, verestert werden. Ferner kann beispielsweise eine oder mehrere Doppelbindungen der Verbindung der Formel (I), (II) oder (III) mit einem Reduktionsmittel reduziert werden, wobei Doppelbindungen zum Beispiel mit $H_2$/Pd reduziert werden.

Weiterhin können die Phenolgruppen der erfindungsgemäßen Verbindungen mit ein-oder mehrwertigen Kationen Chelate bilden. Verbindungen, die chelatbildende Phenolgruppen enthalten, besitzen darüber hinaus einen antioxidativen Effekt (N. Sugihara et al., Journal of Health Science 2001, 47(2), 99-106). Antioxidantien (Oxidationsinhibitoren) sind organische Verbindungen, die unerwünschte, durch Sauerstoff-Einwirkungen bedingte Veränderungen in den zu schützenden Stoffen hemmen oder verhindern. Antioxidantien werden zum Beispiel benötigt in Kunststoffen zum Schutz gegen Alterung, in Fetten zum Schutz gegen Ranzigkeit, in Ölen gegen Verharzung, in Aromastoffen gegen Geruchsverschlechterung, in Lebensmitteln, in Arzneimitteln usw. Die Wirkung der Antioxidantien besteht meist darin, daß sie als Radikalfänger für die bei der Oxidation auftretenden freien Radikale wirken. Die Verbindungen der Formeln (I), (II) und (III) lassen sich daher auch als Chelatbildner und als Antioxidantien verwenden.

Die oben genannten Methoden zur Derivatisierung sind in Lehrbüchern wie Jerry March, Advanced Organic Chemistry, John Wiley & Sons, 4th Edition, 1992, beschrieben. Um Umsetzungen selektiv durchzuführen, kann es

vorteilhaft sein, in an sich bekannter Weise vor der Reaktion geeignete Schutzgruppen einzuführen. Die Schutzgruppen werden nach der Reaktion abgespalten, anschließend wird das Reaktionsprodukt gereinigt.

Die Verbindungen der Formeln (I), (II) und (III) sowie die offensichtlichen chemischen Äquivalente derselben können nach dem Fachmann bekannten Methoden in die entsprechenden pharmakologisch verträglichen Salze überführt werden.

Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Ein Isolat von *Drechslera australiensis*, ST 003360, wurde bei der Deutschen Sammlung von Mikroorganismen und Zellkulturen GmbH (DSM), Mascheroder Weg 1 B, 38124 Braunschweig, Deutschland nach den Regeln des Budapester Vertrages am 28.02.2001 unter der folgenden Nummer hinterlegt: DSM 14093.

Der Stamm *Drechslera australiensis*, ST 003360, DSM 14093, besitzt ein dunkel schwarzes-braunes Mycel und hat keine weiteren charakteristischen Merkmale.

Ein Isolat von eines bisher taxonomisch nicht bestimmten Pilzes, ST 004112, wurde bei der Deutschen Sammlung von Mikroorganismen und Zellkulturen GmbH (DSM), Mascheroder Weg 1 B, 38124 Braunschweig, Deutschland nach den Regeln des Budapester Vertrages unter der folgenden Nummer hinterlegt: DSM 14524.

Der Stamm ST 004112, DSM 14524, ist auf Malz-Agar grau bis schwarz. Isoliert wurde der Stamm aus einer Bodenprobe aus Französisch-Guyana.

Weiterhin betrifft die Erfindung ein Verfahren zur Herstellung der Verbindung der Formel (II), **dadurch gekennzeichnet, daß** der Mikroorganismus ST 003360 (DSM 14093) in einem wässrigen Nährmedium kultiviert wird, eine Verbindung der Formel (II) isoliert und gereinigt wird, und gegebenenfalls in ein offensichtliches chemisches Äquivalent und/oder ein pharmakologisch verträgliches Salz überführt wird.

Weiterhin betrifft die Erfindung ein Verfahren zur Herstellung der Verbindung der Formel (III), **dadurch gekennzeichnet, daß** der Mikroorganismus ST 004112 (DSM 14524) in einem wässrigen Nährmedium kultiviert wird, eine Verbindung der Formel (III) isoliert und gereinigt wird, und gegebenenfalls in ein offensichtliches chemisches Äquivalent oder ein pharmakologisch verträgliches Salz überführt wird.

Weiterhin betrifft die Erfindung ein Verfahren zur Herstellung einer Verbindung der Formel (I), **dadurch gekennzeichnet, daß** a) der Mikroorganismus ST 003360 (DSM 14093) in einem wääsrigen Kulturmedium kultiviert wird, und die Verbindung der Formel (II) isoliert und gereinigt wird, oder daß der Mikroorganismus ST 004112 (DSM 14524) in einem Kulturmedium kultiviert wird, und die Verbindung der Formel (III) isoliert und gereinigt wird, und b) eine Verbindung der Formel (II) oder eine Verbindung der Formel (III) in eine Verbindung der Formel (I) überführt wird, und c) die Verbindung der Formel (I) gegebenenfalls in ein pharmakologisch verträgliches Salz überführt wird.

Der Fermentationsverlauf und die Bildung der erfindungsgemäßen Verbindungen kann entsprechend dem Fachmann bekannten Methoden, wie z. B. durch Testen der biologischen Aktivität in Bioassays oder durch chromatographische Methoden wie Dünnschichtchromatographie (DC) oder Hochleistungsflüssigkeits-Chromatographie (HPLC) verfolgt werden.

In einem Nährmedium, das mindestens eine Kohlenstoff- und Stickstoffquelle sowie die üblichen anorganischen Salze enthält, produziert unter aeroben Bedingungen der Stamm *Drechslera australiensis*, ST 003360, DSM 14093, die erfindungsgemäße Verbindung der Formel (II), und der Stamm ST 004112, DSM 14542, die erfindungsgemäße Verbindung der Formel (III).

Die im folgenden beschriebenen Fermentationsbedingungen gelten für den Stamm *Drechslera australiensis*, ST 003360, DSM 14093, und für den Stamm ST 004112, DSM 14524.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Als organische Salze kann die Nährlösung beispielsweise Chloride, Carbonate, Sulfate oder Phosphate der Alkali- oder Erdalkalimetalle, Eisen, Zink, Kobalt und Mangan enthalten. Als Spurenelemente kann die Nährlösung beispielsweise Molybdän, Kupfer, Nickel oder Selen enthalten.

Die Bildung der erfindungsgemäßen Verbindung (II) verläuft besonders gut in einer Nährlösung, die etwa 0,1 bis 5 %, bevorzugt 0.5% bis 2 % Potatodextrose und 0,2 bis 5 %, bevorzugt 0,5 bis 1 % Hefeextrakt enthält. Die Angaben in Prozent sind jeweils bezogen auf das Gewicht der gesamten Nährlösung.

Die Bildung der erfindungsgemäßen Verbindung (III) verläuft besonders gut in einer Nährlösung, die etwa 0,1 bis 5 %, bevorzugt 0.5% bis 2 % Malzextrakt und 0,2 bis 5 %, bevorzugt 0,5 bis 1 % Hefeextrakt enthält. Die Angaben in Prozent sind jeweils bezogen auf das Gewicht der gesamten Nährlösung.

Die Kultivierung des Mikroorganismus erfolgt aerob, also beispielsweise submers unter Schütteln oder Rühren in Schüttelkolben oder Fermentern, gegebenenfalls unter Einführen von Luft oder Sauerstoff oder auf Festmedien. Sie kann in einem Temperaturbereich von etwa 18 bis 35 °C, vorzugsweise bei etwa 20 bis 30 °C, insbesondere bei 22 bis 28 °C durchgeführt werden. Der pH-Bereich sollte zwischen 4 und 8 liegen, vorzugsweise zwischen 5 und 6. Man kultiviert den Mikroorganismus unter diesen Bedingungen im allgemeinen über einen Zeitraum von 24 bis 300 Stunden, vorzugsweise 36 bis 168 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10 bis 1 :100, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Malz-Hefe-Agar oder Potatodextrose-Agar wachsen läßt. In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

Die erfinderischen Verbindungen kommen sowohl im Mycel als auch im Kulturfiltrat vor. Es ist deshalb zweckmäßig, die Fermentationslösung in das Kulturfiltrat und das Mycel durch Filtration zu trennen und getrennt zu trocknen. Das getrocknete Kulturfiltrat und das getrocknete Mycel werden zweckmäßiger Weise getrennt mit einem organischen Lösungsmittel, zum Beispiel Methanol oder Propanol-2 extrahiert.

Wurde eine Kultur auf Festmedium angelegt, so kommen die erfinderischen Verbindungen ebenfalls sowohl im Mycel als auch im Festagarmedium vor. Zweckmäßigerweise wird die gesamte Kultur lyophilisiert und das Lyophilisat mit einem organischen Lösungsmittel, zum Beispiel Methanol oder Propanol-2 extrahiert.

Die Extraktion kann in einem weiten pH-Bereich durchgeführt werden, es ist jedoch zweckmäßig, im neutralen oder schwach alkalischen Milieu, vorzugsweise zwischen pH 7 und pH 10 zu arbeiten. Der Extrakt kann z. B. im Vakuum konzentriert und getrocknet werden.

Eine Methode der Isolierung der erfindungsgemäßen Verbindungen erfolgt nach dem Trennprinzip der unterschiedlichen Polaritäten in an sich bekannter Weise.

Eine weitere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reversed-Phase Träger, z. B. $RP_8$ und $RP_{18}$, wie sie z. B. im Rahmen der Hochdruckflüssigkeits-Chromatographie (HPLC) allgemein bekannt geworden sind.

Eine weitere Reinigungsmöglichkeit für die erfindungsgemäßen Verbindungen besteht in der Verwendung von sogenannten Normal-Phasen-Chromatographie-Trägern, wie z.B. Kieselgel oder $Al_2O_3$ oder anderen in an sich bekannter Weise.

Ein alternatives Isolierungsverfahren ist die Verwendung von Molekularsieben, wie z. B. Fractogel® TSK HW-40 (Merck, Deutschland) und andere, in an sich bekannter Weise. Es ist darüber hinaus auch möglich, die erfindungsgemäßen Verbindungen aus angereichertem Material durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10. Besonders geeignet ist hierfür die Verwendung von Pufferlösungen, denen man Anteile von organischen Lösungsmitteln hinzugefügt hat.

Es wurde überraschend gefunden, daß die erfindungsgemäßen Verbindungen der Formel (I) Inhibitoren von JNK-3 und CK-1 sind. Tabelle 1 faßt die Wirkdaten der erfinderischen Verbindungen beispielhaft zusammen:

Tabelle 1: Wirkdaten der Verbindungen der Formel (II) und (III)

| Rezeptor | (II) | (III) |
|---|---|---|
| $IC_{50}$ JNK-3 | 1,1 μM | 2,8 μM |
| $IC_{50}$ hCK1ε | 2,9 μM | nicht bestimmt |

Die vorliegende Erfindung betrifft daher auch die Verwendung einer oder mehrerer der erfindungsgemäßen Verbindungen der Formel (I), (II) oder (III) als Arzneimittel, sowie die Verwendung einer oder mehrerer der erfindungsgemäßen Verbindungen der Formel (I), (II) oder (III) zur Herstellung von Arzneimitteln, insbesondere zur Behandlung und/oder zur Prophylaxe von degenerativen Neuropathien, beispielsweise der Alzheimer'schen Krankheit, oder psychischer Erkrankungen, beispielsweise von Depression, Schlafstörungen oder saisonal bedingter affektiver Störungen.

Des weiteren betrifft die vorliegende Erfindung ein Arzneimittel mit einem Gehalt an einer oder mehreren erfindungsgemäßen Verbindungen.

Das besagte Arzneimittel enthaltend eine Verbindung der Formel (I), (II) und/oder (III) wird mit einem oder

mehreren physiologisch geeigneten Hilfsstoffen hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvern (Tabletten, Kapseln einschließlich Mikrokapseln), Salben (Cremes oder Gel), oder Suppositorien verabreicht werden. Als physiologisch geeignete Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Die folgenden Beispiele sollen der näheren Erläuterung der Erfindung dienen, ohne die Breite der Erfindung in irgendeiner Weise begrenzen zu wollen.

Beispiel 1: Herstellung einer Glycerinkultur von *Drechslera australiensis*, ST 003360, DSM 14093.

100 ml Nährlösung (Malzextrakt 2,0 %, Hefeextrakt 0,2 %, Glucose 1,0 % $(NH_4)_2HPO_4$ 0,05 %, pH 6,0) in einem sterilen 300 ml Erlenmeyerkolben wurden mit dem Stamm *Drechslera australiensis*, ST 003360, DSM 14093, beimpft und 7 Tage bei 25 °C und 140 UpM auf einer rotierenden Schüttelmaschine inkubiert. 1,5 ml dieser Kultur wurden anschließend mit 2,5 ml 50 %igem Glycerin verdünnt und bei -135 °C gelagert.

Beispiel 2: Herstellung einer Hauptkultur im Erlenmeyerkolben von *Drechslera australiensis*, ST 003360, DSM 14093.

Ein steriler 300 ml Erlenmeyerkolben mit 100 ml der folgende Nährlösung 2,4 g/l Potatodextrose, 0,2 g/l Hefeextrakt, wurde mit einer auf einem Schrägröhrchen (gleiche Nährlösung, jedoch mit 2 % Agar) gewachsenen Kultur oder mit 1 ml einer Glycerinkultur (s. Beispiel 1) beimpft und auf einer Schüttelmaschine bei 180 UpM und 25 °C inkubiert. Die maximale Produktion des erfindungsgemäßen Drechsleranol der Formel (II) war nach ca. 144 Stunden erreicht. Zum Animpfen von 10 l Fermentern genügte eine 48 bis 96.Stunden alte Submerskultur (Animpfmenge ca. 10 %) aus der gleichen Nährlösung.

Beispiel 3: Herstellung der Verbindung der Formel (II) im 10 l Fermenter.

Ein 10 l Fermenter wurde unter folgenden Bedingungen betrieben:
Nährmedium:
2.4 g/l Potatodextrose
0.2 g/l Hefeestrakt
pH 5.1 (vor der Sterilisation)

| | |
|---|---|
| Inkubationszeit: | 115 Stunden |
| Inkubationstemperatur: | 25 C |
| Rührergeschwindigkeit: | 200 UpM |
| Belüftung: | 15 l/Min |

Durch wiederholte Zugabe von ethanolischer Polyollösung konnte die Schaumbildung unterdrückt werden. Das Produktionsmaximum wurde nach ca. 96 bis 144 Stunden errecht.

Beispiel 4: Isolierung der Verbindung der Formel (II).

Ein Fermentationsansatz aus einem Glas-Fermenter mit einem Nennvolumen von 10 L wurde lyophilisiert und 3x mit jeweils 3 L Methanol extrahiert. Der Methanolextrakt wurde am Vakuum auf ca. 500 ml reduziert und mit Wasser auf einen Methanolgehalt von 10 % verdünnt. Der verdünnte Extrakt (5 L) wurde anschliessend auf eine vorbereitete Glassäule (BPG 100, 4L Innenvolumen, Fa. Pharmacia Biotech) aufgetragen, die mit ca. 0,5 Liter MCl-Gel® CHP-20P Material (Adsorberharz der Fa. Mitsubishi Chemicals, Japan) gefüllt war. Eluiert wurde mit einem Gradienten von 100 % Wasser nach 100 % Acetonitril in 60 min. Der Säulendurchfluß (50 ml / min) wurde fraktioniert aufgefangen (je 50 ml). Sämtliche Fraktionen wurden im JNK-3-Assay getestet und die aktiven Fraktionen (Fraktionen 26-44) zusammengefasst. Konzentrieren am Vakuum und anschliessende Lyophilisierung ergaben 1,21 g eines braunen Pulvers.

Dieses Pulver wurde auf einer LUNA® 10 µ C18 (2) Säule (Größe: 50 mm x 250 mm; Fa. Phenomenex, Deutschland) mit Vorsäule LUNA® 10 µ C18 (2) (Größe: 21.2 mm x 60 mm) aufgetragen und mit einem Gradienten von 0% bis 50 % Acetonitril in 0,1% Ammoniumacetat/Wasser über 50 Minuten chromatographiert. Der Durchfluß des

Elutionsmittels betrug 125 ml/min, die Fraktionsgröße 250 ml. Die Fraktion 17 zeigte im anschliessenden Bioassay die größte Aktivität. Sie wurde lyophilisiert (220 mg) und anschliessend mittels Massenspektrometrie untersucht. Es zeigte sich, daß die Fraktion aus einer einzigen Substanz bestand (Reinheit > 95%).

Beispiel 5: Charakterisierung der Verbindung der Formel (II).

Die physikalisch-chemischen sowie spektroskopischen Eigenschaften der nach Beispiel 4 isolierten Verbindung lassen sich wie folgt zusammenfassen:

Summenformel: $C_{26}H_{24}O_9$
Molekulargewicht: 480
UV-Maxima: 226, 236, 260, 312, 340 nm
$^1H$- und $^{13}C$-NMR: siehe Tabelle 2

Tabelle 2: NMR-chemische Verschiebungen δ (ppm) der Verbindung der Formel (II) in DMSO bei 300 K

| Position | $^1H$ | $^{13}C$ |
|---|---|---|
| 1 | - | 152.57 |
| 2 | - | 118.06 |
| 3 | 7.45 | 129.9 (breit) |
| 4 | - | 124.95 |
| 5 | - | 134.57 |
| 6 | 7.62 | 115.83 |
| 7 | 7.26 | 125.99 |
| 8 | 6.75 | 107.91 |
| 9 | - | 154.76 |
| 10 | - | 115.27 |
| 11 | - | 152.27 |
| 12 | - | 119.16 |
| 13 | 7.38 | 130.13 |
| 14 | 7.30 | 117.58 |
| 15 | - | 135.66 |
| 16 | 7.28 | 118.72 |
| 17 | 7.25 | 126.37 |
| 18 | 6.75 | 108.19 |
| 19 | - | 154.50 |
| 20 | - | 115.15 |
| 21 | 4.62 | 78.4 (breit) |
| 22 | 3.62 | 73.43 |
| 23 | 3.39 | 78.91 |
| 24 | 3.25 | 70.47 |
| 25 | 3.34 | 81.29 |
| 26 | 3.69, 3.45 | 61.37 |

Beispiel 6: Herstellung einer Glycerinkultur von ST 004112, DSM 14524.

100 ml Nährlösung (Malzextrakt 2,0 %, Hefeextrakt 0,2 %, Glucose 1,0 % $(NH_4)_2HPO_4$ 0,05 %, pH 6,0) in einem sterilen 300 ml Erlenmeyerkolben wurden mit dem Stamm DSM 14524 beimpft und 7 Tage bei 25 °C und 140 UpM auf einer rotierenden Schüttelmaschine inkubiert. 1.5 mL dieser Kultur wurden anschließend mit 2,5 ml 50 %igem Glycerin verdünnt und bei -135 °C gelagert.

Beispiel 7: Herstellung einer Hauptkultur von ST 004112, DSM 14524, auf Festmedium (Platten).

50 sterile 25 x 25 $cm^2$ Platten wurden mit jeweils 200 ml einer Nährlösung enthaltend 20 g/l Malzextrakt, 2 g/l Hefeextrakt und 2% Agar und mit einem pH-Wert von 7.0 gegossen. Diese Platten wurden mit 2 ml einer Vorkultur beimpft und bei 25° C inkubiert. Die maximale Produktion der Verbindung der Formel (III) war nach ca. 360 Stunden erreicht.

Beispiel 8: Herstellung einer Hauptkultur im Erlenmeyerkolben von ST 004112, DSM 14524.

Ein steriler 300 ml Erlenmeyerkolben mit 100 ml der folgenden Nährlösung 2,4 g/l Potatodextrose, 0,2 g/l Hefeextrakt, wurde mit einer auf einem Schrägröhrchen (gleiche Nährlösung, jedoch mit 2 % Agar) gewachsenen Kultur oder mit 1 ml einer Glycerinkultur (s. Beispiel 6) beimpft und auf einer Schüttelmaschine bei 180 UpM und 25 °C inkubiert. Die maximale Produktion der Verbindung der Formel (III) war nach ca. 144 Stunden erreicht. Zum Animpfen von 10 l Fermentern genügte eine 48 bis 96 Std. alte Submerskultur (Animpfmenge ca. 10 %) aus der gleichen Nährlösung.

Beispiel 9: Isolierung der Verbindung der Formel (III).

50 Plattenkulturen (20 x 20 cm je Platte) wurden lyophilisiert und 2x mit jeweils 10 L Methanol extrahiert. Der Methanolextrakt wurde am Vakuum auf ca. 500 mL reduziert und mit Wasser auf einen Methanolgehalt von 10 % verdünnt. Der verdünnte Extrakt (5 L) wurde anschliessend auf eine vorbereitete Glassäule (BPG 100, 4L Innenvolumen, Fa. Pharmacia Biotech) aufgetragen, die mit ca. 0,5 Liter MCI-Gel® CHP-20P Material (Adsorberharz der Fa. Mitsubishi Chemicals, Japan) gefüllt war. Eluiert wurde mit einem Gradienten von 100 % Wasser nach 100 % Acetonitril in 30 min. Der Säulendurchfluß (50 mL/min) wurde fraktioniert aufgefangen (je 50 ml). Sämtliche Fraktionen wurden im JNK-3-Assay getestet und die aktiven Fraktionen (Fraktionen 30-44) zusammengefasst. Konzentrieren am Vakuum und anschliessende Lyophilisierung ergab einen braunen gummiartigen Rückstand.

Der Rückstand wurde in Wasser/Acetonitril (1:1) aufgelöst, zentrifugiert und auf einer LUNA® 10 µ C18 (2) Säule (Größe: 21 mm x 250 mm; Fa. Phenomenex, Deutschland) aufgetragen und mit einem Gradienten von 0% bis 100 % Acetonitril in 0,1 % Ammoniumacetat/Wasser über 60 Minuten chromatographiert. Der Durchfluß des Elutionsmittels betrug 33 mL/min, die Fraktionsgröße 33 ml. Die Fraktionen 28-32 zeigten im anschliessenden Bioassay die größte Aktivität. Sie wurden lyophilisiert und anschliessend weiter aufgereinigt. Dazu wurde die Substanz an einer LUNA® 5µ C18 (2) Säule (Größe: 10 mm x 250 mm; Fa. Phenomenex, Deutschland) mit einem Gradienten von 30% bis 60 % Acetonitril in 0,1 % Ammoniumacetat/Wasser über 45 Minuten chromatographiert. Der Durchfluß des Elutionsmittels betrug 6,5 ml/min, die Fraktionsgröße 6,5 ml. Die Fraktionen 18-24 zeigten im anschliessenden Bioassay die größte Aktivität. Nach Gefriertrocknen (Ausbeute: 10 mg) zeigte die anschließende Analyse mittels analytischer HPLC und MS-Spektrometrie, dass es sich um eine einheitliche Verbindung handelte (Reinheit > 95%).

Beispiel 10: Charakterisierung der Verbindung der Formel (III).

Die physikalisch-chemischen sowie spektroskopischen Eigenschaften der nach Beispiel 9 isolierten Verbindung lassen sich wie folgt zusammenfassen:

Summenformel: $C_{20}H_{12}O_3$
Molekulargewicht: 300
UV-Maxima: 226, 236, 260, 312, 340 nm
$^1H$- und $^{13}C$-NMR: siehe Tabelle 3

Tabelle 3: NMR-chemische Verschiebungen δ (ppm) der Verbindung der Formel (III) in MeOD bei 300 K.

| Position | $^{1}H$ | $^{13}C$ |
|---|---|---|
| 1 | ~ | ~ 154.3 (a) |
| 2 | ~ | ~121.3 (a) |
| 3 | 7.46 | 130.63 |
| 4 | 7.31 | 119.55 |
| 5 | ~ | 137.60 |
| 6 | 7.24 | 119.55 |
| 7 | 7.22 | 127.52 |
| 8 | 6.69 | 109.20 |
| 9 | ~ | ~157.0 |
| 10 | ~ | ~117.5 (a) |

(a) Für diese Kohlenstoffatome wird im $^{13}C$-Spektrum kein (oder ein extrem breites) Signal beobachtet. Die chemischen Verschiebungen wurden daher anhand von Korrelationen im HMBC-Spektrum bestimmt.

Beispiel 11: Nachweis der Wirksamkeit der Verbindungen der Formeln (II) und (III) im JNK-3-Assay

Der Assay wird auf einem CyBio-Pipettiersystem im 384-Lochplattenformat durchgeführt. In einem Endvolumen von 30 µl enthält der Assay 10 µl Probe (Extrakt oder Reinsubstanz, beispielsweise eine Verbindung der Formel (II) oder der Formel (III)) in 3% DMSO, 10 µl einer Enzym/Substratmischung (JNK-3 /GST-ATF2) und 10 µl ATP. Nach 20 Minuten Inkubation bei 37°C werden 50 µl der HTRF Antibody-Mischung (XL665-anti-GST/ (Eu)cryptat anti-P-ATF2) zugegeben. Nach 120 Minuten bei Raumtemperatur wird die Signalemission des Energietransfers und von Europium bei 665 und 615 nm gemessen, nachdem die Proben bei 340 nm in einem Victor[2] (Fa. WALLAC) angeregt wurden.

Puffer I für das Verdünnen von JNK3, GST-ATF2, ATP:

| | |
|---|---|
| 25 mM | HEPES, pH 7.5 |
| 100 µM | $MgCl_2$ |
| 0.03% | TRITON X 100 |
| 10 mM | DTT |
| 5 % | Glycerol |

Puffer II für das Verdünnen von der HTRF Reagenzien:

| | |
|---|---|
| 100 mM | HEPES, pH 7.0 |
| 100 mM | KF |
| 133 mM | EDTA |
| 1 g/l | BSA |

| Reagenzien: | Lieferant: | Endkonzentration: |
|---|---|---|
| JNK3 Kinase | Biotech, Vitry | 8 ng / well |
| GST-ATF2 | Biotech, Vitry | 88 ng / well |
| ATP | Sigma, A7699 | 15 µM |
| Anti-GST-XL665 | CisBio | 125 ng / well |
| Anti-P-ATF2-(Eu)cryptate | NEB/CisBio | 6 ng / well |

Jede Platte enthält 16 Positiv-Kontrollen (maximaler Energietransfer, Puffer I anstelle von Proben), 8 Blank-Kontrollen (minimaler Energietransfer, Puffer II anstelle von ATP) und 8 Löcher, die EDTA 200 µM enthalten.

Die Ergebnisse werden wie folgt berechnet:

Zunächst wird das Signalverhältnis = (Intensität(665nm) / Intensität(615nm)) ermittelt. Anschliessend wird eine Blank-Korrektur durchgeführt bei der man die folgende Formel verwendet:

$$\text{Delta F (\%)} = (\text{Verhältnis(Probe)} - \text{Verhältnis(min)}) / (\text{Verhältnis(min)} \times 100)$$

Anschliessend läßt sich die Aktivität der Proben folgendermaßen berechnen:

$$\text{Inhibition (\%)} = 100 \times [\,1 - (\text{Delta F(Probe)} / \text{Delta F(max)})$$

Beispiel 12: Nachweis der Wirksamkeit der Verbindung der Formel (II) im hCK1ε-Assay

Der Assay wird auf einem Jobi-Well (CyBio) und Biomek 2000 Pipettiersystem im 384-Lochplattenformat durchgeführt. Die 384-Lochplatten werden mit 50 μl pro well einer Caseinlösung der Konzentration 100 μg/ml in Beschichtungspuffer beschichtet (entspricht 5 μg Casein pro well, Casein Fa. Sigma) und wird über Nacht bei 4°C gelagert. Anschließend wird viermal mit 90 μl der Waschlösung 1 (50 mM HEPES pH 7,4 und 150 mM NaCl) gewaschen. Die Reaktion wird in einem Endvolumen von 50 μl durchgeführt. Dabei wird auf die beschichteten Platten jeweils 10 μl verdünnter Naturstoffextrakt, beispielsweise eine Verbindung der Formel (II) oder eine Verbindung der Formel (III), 20 μl hCK1ε-Enzymlösung (entspricht 29 ng Casein pro well) und 20 μl ATP-Lösung (Endkonzentration: 0,4 μCi $^{33}$P-γ-ATP radioaktiv markiertes ("heisses") und 0,4 μM kaltes ATP pro well) pipettiert Anschließend werden die Platten bei 37 °C für eine Stunde inkubiert. Die Platten werden dann viermal mit 75 μl Waschlösung 2 (Phosphorsäure 3 %) gewaschen und in einem MicroBeta Trilux Counter (Fa. WALLAC) für 30 Sekunden vermessen.

hCK1ε-Enzymlösung: 1,45 μg rekombinantes hCK1ε pro mL Kinase-Puffer

Kinase-Puffer:

50 M HEPES pH 7,4
10 mM $MgCl_2$
0,25 mM DTT
0,6 mM EGTA

Beschichtungspuffer:

27,5 mM $Na_2CO_3$
22,5 mM $NaHCO_3$ (pH 9,6)
in 0,9 % NaCl

ATP-Lösung: 20 μCi/ml $^{33}$P-γ-ATP und 1 μM kaltes ATP

Auf jeder Platte werden 16 Löcher dazu verwendet, um die totale Enzymaktivität zu bestimmen (ohne Inhibitorzusatz) und weitere 16 ohne Enzymzusatz, um die nichtspezifische Reaktion zu bestimmen.

Die Inhibierung einer Probe läßt sich nach der folgenden Formel berechnen:

$$[1-(\text{CPM(Probe)} - \text{CPM(nichtspez.)}) / (\text{CPM(Enzym Konzentration)} - \text{CPM (nichtspez.)})] \times 100\ (\%)$$

CPM = counts per minute

Die Ergebnisse des JNK-3 und hCK1ε-Assays sind in Tabelle 1 (*vide supra*) zusammengefaßt.

### Patentansprüche

1. Verbindung der Formel (I)

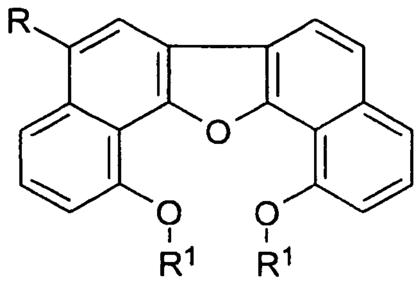

(I)

wobei
R H, oder eine Gruppe der Formel $-(CH(OR^2))_5-CH_2-OR^2$, und
$R^1$ und $R^2$ unabhängig voneinander
1.0 H oder
2.0 eine $C_1$-$C_6$-Alkyl-, $C_2$-$C_6$-Alkenyl-, $C_2$-$C_6$-Alkinyl- oder $C_5$-$C_{10}$-Aryl-Gruppe bedeuten, worin Alkyl, Alkenyl und Alkinyl geradkettig oder verzweigt sind, und worin die Gruppen gegebenenfalls ein- oder zweifach substituiert sind durch:

2.1 -OH,
2.2 =O,
2.3 -O-$C_1$-$C_6$-Alkyl, worin Alkyl geradkettig oder verzweigt ist,
2.4 -O-$C_2$-$C_6$-Alkenyl, worin Alkenyl geradkettig oder verzweigt ist,
2.5 -Aryl,
2.6 -NH-$C_1$-$C_6$-Alkyl, worin Alkyl geradkettig oder verzweigt ist,
2.7 -NH-$C_2$-$C_6$-Alkenyl, worin Alkenyl geradkettig oder verzweigt ist,
2.8-$NH_2$ oder
2.9 Halogen,

worin die Substituenten 2.3 bis 2.7 weiter substituiert sein können durch -CN, - Amid oder -Oxim-Funktionen, oder eine stereoisomere Form der Verbindung der Formel (I) oder ein physiologisch verträgliches Salz der Verbindung der Formel (I) oder ein physiologisch verträgliches Salz einer stereoisomeren Form einer Verbindung der Formel (I).

**2.** Verbindung der Formel (I) gemäß Anspruch 1, wobei
R H oder eine Gruppe der Formel $-(CH(OR^2))_5-CH_2-OR^2$ ist,
$R^1$ und $R^2$ unabhängig voneinander H oder $C_1$-$C_6$-Alkyl sind, worin $C_1$-$C_6$-Alkyl geradkettig oder verzweigt ist und gegebenenfalls ein- oder zweifach durch die Reste 2.1 bis 2.9 substituiert ist.

**3.** Verbindung der Formel (I) gemäß Anspruch 1, **dadurch gekennzeichnet, daß** sie die Formel (II) hat

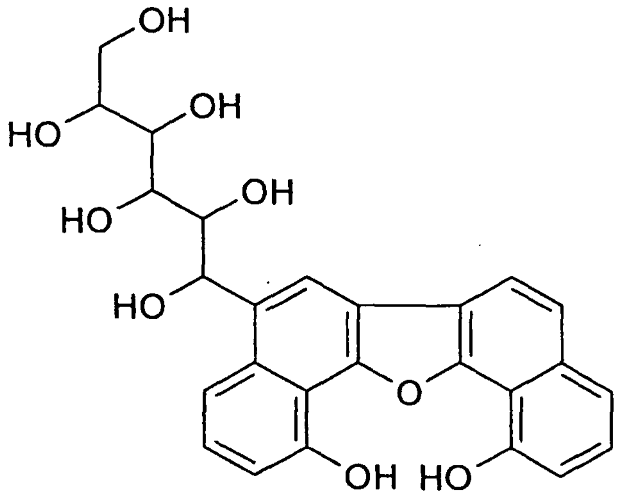

(II)

oder eine stereoisomere Form der Verbindung der Formel (II) oder ein physiologisch verträgliches Salz der Verbindung der Formel (II) oder oder ein physiologisch verträgliches Salz einer stereoisomeren Form einer Verbindung der Formel (II).

4. Verbindung der Formel (I) gemäß Anspruch 1, **dadurch gekennzeichnet, daß** sie die Formel (III) hat

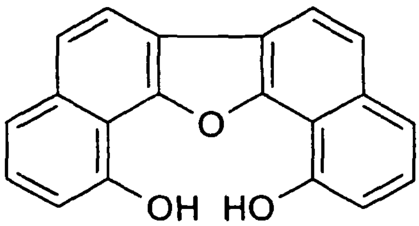

(III)

oder eine stereoisomere Form der Verbindung der Formel (III) oder ein physiologisch verträgliches Salz der Verbindung der Formel (III) oder oder ein physiologisch verträgliches Salz einer stereoisomeren Form einer Verbindung der Formel (III).

5. Verfahren zur Herstellung der Verbindung der Formel (II) gemäß Anspruch 3 oder pharmakologisch verträglichen Salzes einer Verbindung der Formel (II), **dadurch gekennzeichnet, daß**

   a) der Mikroorganismus ST 003360 (DSM 14093) kultiviert wird,
   b) eine Verbindung der Formel (II) isoliert und gereinigt wird, und
   c) die Verbindung der Formel (II) gegebenfalls in ein pharmakologisch verträgliches Salz überführt wird.

6. Verfahren zur Herstellung der Verbindung der Formel (III) gemäß Anspruch 4 oder eines pharmakologisch verträglichen Salzes einer Verbindung der Formel (III), **dadurch gekennzeichnet, daß**

   a) der Mikroorganismus ST 004112 (DSM 14524) kultiviert wird,
   b) eine Verbindung der Formel (III) isoliert und gereinigt wird, und
   c) die Verbindung der Formel (III) gegebenfalls in ein offensichtliches chemisches Äquivalent und/oder ein pharmakologisch verträgliches Salz überführt wird.

7. Verfahren zur Herstellung einer Verbindung der Formel (I) gemäß Anspruch 1 oder eines pharmakologisch verträglichen Salzes einer Verbindung der Formel (I), **dadurch gekennzeichnet, daß**

a) der Mikroorganismus ST 003360 (DSM 14093) in einem Kulturmedium kultiviert wird, und die Verbindung der Formel (II) isoliert und gereinigt wird,
oder daß der Mikroorganismus ST 004112 (DSM 14524) in einem Kulturmedium kultiviert wird, und die Verbindung der Formel (III) isoliert und gereinigt wird, und
b) eine Verbindung der Formel (II) oder eine Verbindung der Formel (III) in eine Verbindung der Formel (I) überführt wird, und
c) die Verbindung der Formel (I) gegebenenfalls in ein pharmakologisch verträgliches Salz überführt wird.

**8.** Verwendung einer Verbindung gemäß einem der Ansprüche 1 bis 4 zur Herstellung eines Arzneimittels.

**9.** Verwendung einer Verbindung gemäß einem der Ansprüche 1 bis 4 zur Herstellung eines Arzneimittels zur Therapie und/oder Prophylaxe von degenerativen Neuropathien.

**10.** Verwendung einer Verbindung gemäß einem der Ansprüche 1 bis 4 zur Herstellung eines Arzneimittels zur Therapie und/oder Prophylaxe der Alzheimer'schen Krankheit.

**11.** Verwendung einer Verbindung gemäß einem der Ansprüche 1 bis 4 zur Herstellung eines Arzneimittels zur Therapie und/oder Prophylaxe von psychischen Erkrankungen.

**12.** Verwendung einer Verbindung gemäß einem der Ansprüche 1 bis 4 zur Herstellung eines Arzneimittels zur Therapie und/oder Prophylaxe von Depressionen, Schlafstörungen oder saisonal bedingten affektiven Störungen.

**13.** Verwendung einer Verbindung gemäß einem der Ansprüche 1 bis 4 als Chelatbildner oder Antioxidant.

**14.** Arzneimittel enthaltend mit einem Gehalt an mindestens einer Verbindung gemäß einem oder mehreren der Ansprüche 1 bis 9 und einem oder mehreren physiologisch geeigneten Hilfsstoffen.

**15.** Mikroorganismen-Stamm *Drechslera australiensis* (DSM 14093).

**16.** Mikroorganismen-Stamm DSM 14524.

**Claims**

**1.** A compound of the formula (I)

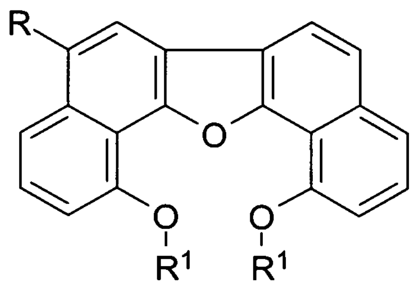

(I)

where
R is H, or a group of the formula $-(CH(OR^2))_5-CH_2-OR^2$, and
$R^1$ and $R^2$ independently of one another are 1.0 H or
2.0 a $C_1$-$C_6$-alkyl, $C_2$-$C_6$-alkenyl, $C_2$-$C_6$-alkynyl or $C_5$-$C_{10}$-aryl group, in which alkyl, alkenyl and alkynyl are straight-chain or branched, and in which the groups are optionally mono- or disubstituted by:

2.1 -OH,
2.2 =O,
2.3 -O-$C_1$-$C_6$-alkyl, in which alkyl is straight-chain or branched,
2.4 -O-$C_2$-$C_6$-alkenyl, in which alkenyl is straight-chain or branched,

2.5 -aryl,
2.6 -NH-$C_1$-$C_6$-alkyl, in which alkyl is straight-chain or branched,
2.7 -NH-$C_2$-$C_6$-alkenyl, in which alkenyl is straight-chain or branched,
2.8-$NH_2$ or
2.9 halogen,
in which the substituents 2.3 to 2.7 can further be substituted by -CN, -amide or -oxime functions, or a stereoisomeric form of the compound of the formula (I) or a physiologically tolerable salt of the compound of the formula (I) or a physiologically tolerable salt of a stereoisomeric form of a compound of the formula (I).

**2.** A compound of the formula (I) as claimed in claim 1, where R is H or a group of the formula -(CH($OR^2$))$_5$-$CH_2$-$OR^2$, $R^1$ and $R^2$ independently of one another are H or $C_1$-$C_6$-alkyl, in which $C_1$-$C_6$-alkyl is straight-chain or branched and optionally mono- or disubstituted by the radicals 2.1 to 2.9.

**3.** A compound of the formula (I) as claimed in claim 1, **characterized in that** it has the formula (II)

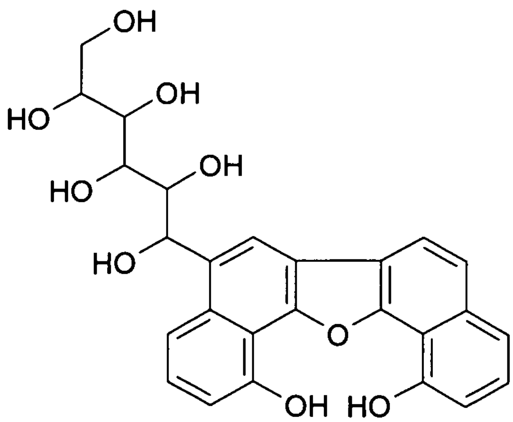

(II)

or a stereoisomeric form of the compound of the formula (II) or a physiologically tolerable salt of the compound of the formula (II) or a physiologically tolerable salt of a stereoisomeric form of a compound of the formula (II).

**4.** A compound of the formula (I) as claimed in claim 1, **characterized in that** it has the formula (III)

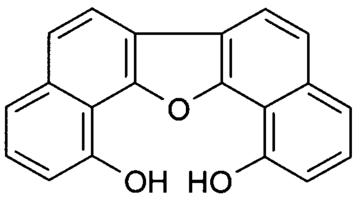

(III)

or a stereoisomeric form of the compound of the formula (III) or a physiologically tolerable salt of the compound of the formula (III) or a physiologically tolerable salt of a stereoisomeric form of a compound of the formula (III).

**5.** A process for the preparation of the compound of the formula (II) as claimed in claim 3 or pharmacologically tolerable salt of a compound of the formula (II), **characterized in that** it comprises

a) culturing the microorganism ST 003360 (DSM 14093),
b) isolating and purifying a compound of the formula (II), and
c) converting the compound of the formula (II), if appropriate, into a pharmacologically tolerable salt.

**6.** A process for the preparation of the compound of the formula (III) as claimed in claim 4 or of a pharmacologically tolerable salt of a compound of the formula (III), **characterized in that** it comprises

a) culturing the microorganism ST 004112 (DSM 14524),

b) isolating and purifying a compound of the formula (III), and
c) converting the compound of the formula (III), if appropriate, into an obvious chemical equivalent and/or a pharmacologically tolerable salt.

**7.** A process for the preparation of a compound of the formula (I) as claimed in claim 1 or of a pharmacologically tolerable salt of a compound of the formula (I), **characterized in that** it comprises

a) culturing the microorganism ST 003360 (DSM 14093) in a culture medium, and
isolating and purifying the compound of the formula (II) ,
or culturing the microorganism ST 004112 (DSM 14524) in a culture medium, and isolating and purifying the compound of the formula (III), and
b) converting a compound of the formula (II) or a compound of the formula (III) into a compound of the formula (I), and
c) converting the compound of the formula (I), if appropriate, into a pharmacologically tolerable salt.

**8.** The use of a compound as claimed in one of claims 1 to 4 for the production of a pharmaceutical.

**9.** The use of a compound as claimed in one of claims 1 to 4 for the production of a pharmaceutical for the therapy and/or prophylaxis of degenerative neuropathies.

**10.** The use of a compound as claimed in one of claims 1 to 4 for the production of a pharmaceutical for the therapy and/or prophylaxis of Alzheimer’s disease.

**11.** The use of a compound as claimed in one of claims 1 to 4 for the production of a pharmaceutical for the therapy and/or prophylaxis of psychological disorders.

**12.** The use of a compound as claimed in one of claims 1 to 4 for the production of a pharmaceutical for the therapy and/or prophylaxis of depressions, sleep disturbances or seasonally related affective disorders.

**13.** The use of a compound as claimed in one of claims 1 to 4 as a chelating agent or antioxidant.

**14.** A pharmaceutical containing at least one compound as claimed in one or more of claims 1 to 4 and one or more physiologically suitable excipients.

**15.** The microorganism strain *Drechslera australiensis* (DSM 14093).

**16.** The microorganism strain DSM 14524.

## Revendications

**1.** Composé de formule (I)

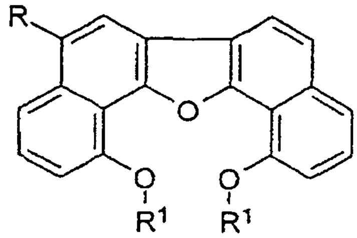

(I)

où
R signifie H, ou un groupe de formule $-(CH(OR^2))_5-CH_2-OR^2$, et
$R^1$ et $R^2$ signifient, indépendamment l’un de l’autre 1.0 H ou
2.0 un groupe $C_1$-$C_6$-alkyle, $C_2$-$C_6$-alcényle, $C_2$-$C_6$-alcynyle ou $C_5$-$C_{10}$-aryle, où alkyle, alcényle et alcynyle sont linéaires ou ramifiés et où les groupes sont le cas échéant monosubstitués ou disubstitués par

2.1 -OH,
2.2 =O,
2.3 -O-$C_1$-$C_6$-alkyle, où alkyle est linéaire ou ramifié,
2.4 -O-$C_2$-$C_6$-alcényle, où alcényle est linéaire ou ramifié,
2.5 -aryle,
2.6 -NH-$C_1$-$C_6$-alkyle, où alkyle est linéaire ou ramifié,
2.7 -NH-$C_2$-$C_6$-alcényle, où alcényle est linéaire ou ramifié,
2.8 -$NH_2$ ou
2.9 halogène,
où les substituants 2.3 à 2.7 peuvent en outre être substitués par des fonctions -CN, -amide ou -oxime,
ou une forme stéréo-isomère du composé de formule (I) ou un sel physiologiquement acceptable du composé de formule (I) ou un sel physiologiquement acceptable d'une forme stéréo-isomère d'un composé de formule (I).

**2.** Composé de formule (I) selon la revendication 1, où
R signifie H ou un groupe de formule -$(CH(OR^2))_5$-$CH_2$-$OR^2$,
$R^1$ et $R^2$ représentent, indépendamment l'un de l'autre, H ou $C_1$-$C_6$-alkyle, où $C_1$-$C_6$-alkyle est linéaire ou ramifié et le cas échéant monosubstitué ou disubstitué par les radicaux 2.1 à 2.9.

**3.** Composé de formule (I) selon la revendication 1, **caractérisé en ce qu'**il présente la formule (II)

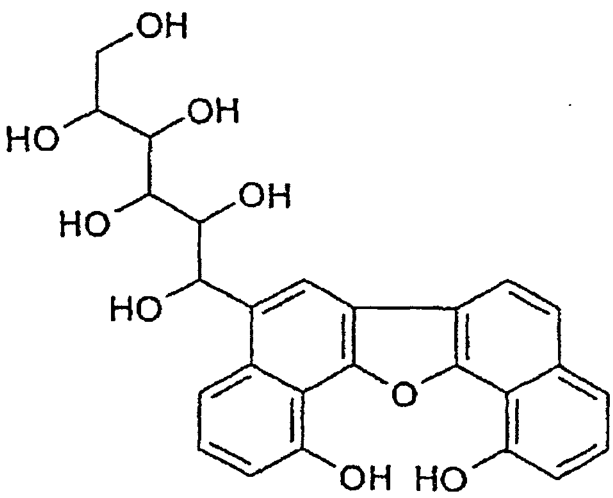

(II)

ou une forme stéréo-isomère du composé de formule (II) ou un sel physiologiquement acceptable du composé de formule (II) ou un sel physiologiquement acceptable d'une forme stéréo-isomère du composé de formule (II).

**4.** Composé de formule (I) selon la revendication 1, **caractérisé en ce qu'**il présente la formule (III)

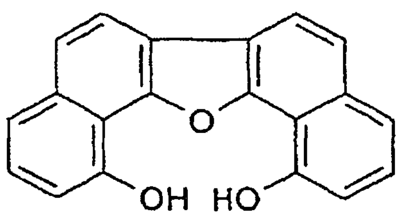

(III)

ou une forme stéréo-isomère du composé de formule (III) ou un sel physiologiquement acceptable du composé de formule (III) ou un sel physiologiquement acceptable d'une forme stéréo-isomère d'un composé de formule (III).

**5.** Procédé pour la préparation du composé de formule (II) selon la revendication 3 ou du sel pharmacologiquement acceptable d'un composé de formule (II), **caractérisé en ce qu'**on

a) cultive le microorganisme ST 003360 (DSM 14093),
b) isole et purifie un composé de formule (II), et
c) transforme le composé de formule (II) le cas échéant en un sel pharmacologiquement acceptable.

**6.** Procédé pour la préparation du composé de formule (III) selon la revendication 4 ou d'un sel pharmacologiquement acceptable d'un composé de formule (III), **caractérisé en ce qu'**on

a) cultive le microorganisme ST 004112 (DSM 14524),
b) isole et purifie un composé de formule (III), et
c) transforme le composé de formule (III) le cas échéant en un équivalent chimique manifeste et/ou en un sel pharmacologiquement acceptable.

**7.** Procédé pour la préparation d'un composé de formule (I) selon la revendication 1 ou d'un sel pharmacologiquement acceptable d'un composé de formule (I), **caractérisé en ce qu'**on

a) cultive le microorganisme ST 003360 (DSM 14093) dans un milieu de culture, et
isole et purifie le composé de formule (II),
ou cultive le microorganisme ST 004112 (DSM 14524) dans un milieu de culture, et isole et purifie le composé de formule (III), et
b) transforme un composé de formule (II) ou un composé de formule (III) en un composé de formule (I), et
c) transforme le composé de formule (I) le cas échéant en un sel pharmacologiquement acceptable.

**8.** Utilisation d'un composé selon l'une quelconque des revendications 1 à 4 pour la préparation d'un médicament.

**9.** Utilisation d'un composé selon l'une quelconque des revendications 1 à 4 pour la préparation d'un médicament destiné à la thérapie et/ou la prophylaxie de neuropathies de dégénérescence.

**10.** Utilisation d'un composé selon l'une quelconque des revendications 1 à 4 pour la préparation d'un médicament destiné à la thérapie et/ou la prophylaxie de la maladie d'Alzheimer.

**11.** Utilisation d'un composé selon l'une quelconque des revendications 1 à 4 pour la préparation d'un médicament destiné à la thérapie et/ou la prophylaxie de maladies psychiques.

**12.** Utilisation d'un composé selon l'une quelconque des revendications 1 à 4 pour la préparation d'un médicament destiné à la thérapie et/ou la prophylaxie de dépressions, de troubles du sommeil ou de troubles affectifs liés aux saisons.

**13.** Utilisation d'un composé selon l'une quelconque des revendications 1 à 4 comme chélatant ou antioxydant.

**14.** Médicament contenant une teneur en au moins un composé selon l'une ou plusieurs des revendications 1 à 4 et un ou plusieurs adjuvants physiologiquement appropriés.

**15.** Souche de microorganismes *Drechslera australiensis* (DSM 14093).

**16.** Souche de microorganismes DSM 14524.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Kumagae et al.** *Mol. Brain Res.,* 1999, vol. 67 (1), 10-7
- **Keesler et al.** *Neuroreport,* 2000, vol. 11 (5), 951-955
- **Souetre E. et al.** *Annales medico-physiologiques,* 1985, vol. 143 (9), 845-870
- **W. Cocker et al.** *Journal of the Chemical Society,* 1953, 2355-2362
- **Sugihara et al.** *Journal of Health Science,* 2001, vol. 47 (2), 99-106
- **Jerry March.** Advanced Organic Chemistry. John Wiley & Sons, 1992
- Remingtons Pharmaceutical Sciences. 1985, 1418